# EUROPÄISCHE PATENTSCHRIFT

(11) **EP 0 615 446 B1**
(45) Veröffentlichungstag und Bekanntmachung des Hinweises auf die Patenterteilung: **15.11.1995**
(21) Anmeldenummer: 92924551.2
(22) Anmeldetag: 04.12.1992
(51) Int. Cl.: A61K 9/51, A61K 9/10, A61K 47/42, A61K 31/44, A61K 9/20, A61K 9/50

(54) **EIN DIHYDROPYRIDINDERIVAT IN NANOSOLFORM ENTHALTENDES RETARD-ARZNEIMITTEL UND SEINE HERSTELLUNG**
SLOW-RELEASE MEDICAMENT CONTAINING A DIHYDROPYRIDINE DERIVATE AS A NANOSOL AND ITS PREPARATION
MEDICAMENT A EFFET RETARD CONTENANT UN DERIVE DE DIHYDROPYRIDINE SOUS FORME DE NANOSOL ET SA PREPARATION

(30) Priorität: 05.12.1991 DE 4140194
(43) Veröffentlichungstag der Anmeldung: 21.09.1994
(73) Patentinhaber: ALFATEC-PHARMA GmbH, 69120 Heidelberg (DE)
(72) Erfinder: WUNDERLICH, Jens-Christian, D-6900 Heidelberg (DE); SCHICK, Ursula, D-6908 Wiesloch (DE); WERRY, Jürgen, D-6700 Ludwigshafen (DE); FREIDENREICH, Jürgen, D-6905 Schriesheim (DE)
(74) Vertreter: Fürniss, Peter, Dr.
(86) Internationale Anmeldenummer: DE9201014
(87) Internationale Veröffentlichungsnummer: WO9310770

(56) Entgegenhaltungen:
- EP-A- 0 167 825
- EP-A- 0 200 068
- EP-A- 0 282 020
- EP-A- 0 349 428
- AU-A- 495 261
- FR-A- 2 608 427

## Beschreibung

Die Erfindung betrifft ein Arzneimittel, das ein pharmakologisch wirksames Dihydropyridinderivat in Form eines pharmazeutisch applizierbarten Nanosols enthält, insbesondere Retardformen hiervon, sowie ein Verfahren zu seiner Herstellung und seine Verwendung.

Herz-Kreislauferkrankungen gehören weltweit zu den häufigsten Krankheiten in den hochentwickelten Industrieländern. Die Calciumantagonisten, allen voran die Gruppe der Dihydropyridinderivate mit dem bekanntesten Vertreter Nifedipin, gehören seit den 70er und 80er Jahren zu den umsatzstärksten Arzneimitteln am Arzneimittelmarkt überhaupt. Nach erfolgreicher Einführung des Nifedipins 1,4-Dihydro-2,6-dimethyl-4-(2-nitrophenyl)-3,5 pyridincarbonsäuredimethylester, C₁₇H₁₈N₂O₆, wurden eine Reihe von Strukturverwandten entwikkelt, z.B. Nitrendipin 1,4-Dihydro-2,6-dimethyl-4-(3-nitrophenyl)-3,5 pyridincarbonsäure-ethyl-methylester, C₁₈H₂₀N₂0₆ und Nisoldipin Isobutyl-methyl-1,4-dihydro-2,6-dimethyl-4-(2-nitrophenyl)pyridin-3,5-dicarboxylat, C₂₀H₂₄N₂0₆, die man als Dihydropyridine der zweiten Generation bezeichnet. Retardformulierungen für diese Dihydropyridine, besonders für Nifedipin sind vielfach vorgeschlagen worden.

Obwohl auf dem derzeitigen Arzneimittelmarkt verschiedenste galenische Entwicklungen peroraler Retardzubereitungen angeboten werden, ist es für Dihydropyridinderivate, insbesondere Nifedipin, bis heute noch nicht gelungen, den in-vivo Resorptionsprozeß des aus einer entsprechenden Arzneiform primär freigegebenen Wirkstoffs so optimal an die physiologischen Gegebenheiten (pH-Verhältnisse im Gastrointestinaltrakt, gastrointestinale Verweilzeiten von Formlingen, spezifische Resorptionsfenster für bestimmte Wirkstoffe) anzupassen, daß die Hauptanforderungen an eine Retardarzneiform erfüllt werden.

Retardierung eines Wirkstoffs in einer pharmazeutischen Darreichungsform (in engerem Sinne Peroralia) ist dann erwünscht, wenn die biologische Halbwertszeit dieses Wirkstoffs kurz ist (in der Regel unterhalb von 10-12 h). Dies trifft insbesondere für die Gruppe der Dihydropyridine zu, deren durchschnittliche Eliminationshalbwertszeiten zwischen 2 und 8 Stunden liegen, wobei z.B. bei Nifedipin die schnelle Elimination einem ausgeprägten First-pass-Metabolismus unterliegt . Durch lang anhaltende Freigabe aus der Arzneiform im Organismus erhofft man sich verschiedene Vorteile:
1) Verbesserte Wirkung
   Möglichst genaue Einstellung von Plasmaspiegeln im therapeutischen Niveau soll einerseits Plasmaspiegelschwankungen vermindern, andererseits Nebenwirkungen (u.U. toxisch) vermeiden.
2) Verlängerte Wirkung
   damit verbunden ist analog eine entsprechende Reduktion der Einnahmehäufigkeit und dadurch eine entscheidende Erhöhung der Patienten-Compliance.
3) Verminderung der insgesamt verabreichten Arzneistoffdosis bei Erzielung vergleichbarer Wirkung gegenüber der mehrfachen Einzelgabe.

Der galenische Aufbau von bekannten Retardarzneiformen ist i.a. so konzipiert, daß die Wirkstofffreigabe im Organismus den geschwindigkeitsbestimmenden Schritt im Freigabe-Resorptions-Geschehen darstellt. Aus einem Depot soll der Wirkstoff verzögert und möglichst gleichmäßig (idealerweise konstant = Kinetik nullter Ordnung) freigegeben werden, um einen konstanten Übergang in die Biophase zu erreichen. Es ist jedoch bisher noch so, daß besagter Wirkstoff nach seiner Freisetzung weitestgehend unkontrollierbar seinem Schicksal überlassen bleibt, d.h. physiologische Einflußgrößen im Gastrointestinaltrakt bleiben unberücksichtigt.

Dies trifft besonders für die schwer wasserlöslichen Wirkstoffe aus der Gruppe der Dihydropyridine, insbesondere Nifedipin zu, denen daher eine problematische Bioverfügbarkeit zugeschrieben werden muß. Trotz einer Vielzahl von verschiedenen galenischen Entwicklungen scheint es noch nicht gelungen zu sein, eine Formulierung für diese Wirkstoffgruppe zu entwickeln, die alle Anforderungen an eine effiziente Therapie bei Herz-Kreislauferkrankungen erfüllt.

Es ist bereits vorgeschlagen worden, Dihydropyridinderivate, insbesondere Nifedipin in vielfältig modulierbarer Art und Weise über einen bestimmten Zeitraum aus geeigneten Arzneiformen verzögert freizusetzen (z.B. EP-Appl. 0 232 155 und 0 274 176). Viele Nifedipin-Retardpräparate beruhen dabei beispielsweise auf folgendem Prinzip: Der Wirkstoff wird in verschiedenen Polymermatrices (z.B. Polyethylenglykol oder Polyvinylpyrrolidon) eingebettet, die in-vivo als Lösungsvermittler für das schwer wasserlösliche Nifedipin wirken sollen, um die Resorption überhaupt in ausreichendem Maße zu ermöglichen.

Es ist aber nicht anzunehmen, daß üblicherweise in-vitro gemessene Freigabe-Zeit-Profile auch mit der in-vivo Resorption dieser Stoffe in Korrelation gebracht werden können. Daher gelingt es nur ungenügend, Dihydropyridinderivate, insbesondere Nifedipin unmittelbar in resorptionsfähiger Form an den verschiedenen Resorptionsorten des Gastrointestinaltrakts zur Verfügung zu stellen. Die Folge ist nun einerseits, daß eine konstante Anflutung des Wirkstoffs in der Biophase nach Applikation einer Retardformulierung nicht sichergestellt werden kann und andererseits ein Wirkeintritt zeitlich nicht oder nur sehr schwierig vorherbestimmbar wird.

Das oben gesagte wird besonders deutlich, wenn man die gastrointestinale Passage einer konstant (nullter Ordnung) freisetzenden, Nifedipin enthaltenden Retardarzneiform verfolgt. Diese ist beispielsweise bei einer neueren galenischen Entwicklung, dem nach dem Prinzip einer osmotischen Pumpe arbeitenden, sogenannten OROS-System verwirklicht.

Nach heute allgemein akzeptierter Theorie verläuft die Wirkstoff-Resorption überwiegend nach den Gesetzen der passiven Diffusion, d.h. nur gelöste und gleichzeitig undissoziierte Wirkstoffmoleküle werden resorbiert. Für Nifedipin (genauso für andere Dihydropyridine), die bekanntermaßen schwer wasserlösliche Neutralstoffe darstellen, ist daher eine nennenswerte Resorption grundsätzlich nicht zu erwarten. Durch bekannte galenische Maßnahmen zur Löslichkeitserhöhung wie z.B. Solubilisation oder Komplexbildung, kann diese jedoch in einem beschränkten Umfang verbessert werden. Gleiches gilt für das Mikronisieren von Wirkstoffen, wobei eine höhere Lösungsgeschwindigkeit durch Vergrößerung der effektiven Stoffoberfläche erzwungen werden soll. Es ist aber stets zu beachten, daß löslich gemachte Anteile rekristallisieren können, was besonders zu Schleimhautirritationen führen kann.

Untersuchungen haben gezeigt, daß trotz dieser galenischen Maßnahmen die Nifedipinresorption im Magen nur unzureichend abläuft. Für ein entsprechendes, konventionelles Retardpräparat bedeutet dies wiederum eine erst relativ spät nach der Applikation einsetzende Wirkung.

Schwankende Magenverweilzeiten von peroralen Retardarzneiformen verhindern zusätzlich, daß eine Wirkstoffdosis einen günstigeren Resorptionsort erreicht. So ist eine Schwankungsbreite von 0,5-10 h keine Seltenheit. Nahrungsaufnahme sowie Art und Menge der Nahrung, die Größe und Dichte der Arzneiform etc. haben entscheidenden Einfluß. Die Freisetzung des Wirkstoffs läuft aber in dieser Zeit kontinuierlich weiter. Das verdeutlicht insbesondere, daß eine ausreichende Anflutung in der Biophase nicht erwartet werden kann, es resultieren subtherapeutische Plasmaspiegel. Gleichzeitig steigt das Risiko u.U. toxischer gastrointestinaler Nebenwirkungen erheblich an. Auch in den verschiedenen Dünndarmabschnitten ergibt sich ein ähnliches Bild wie im Magen.

Erst nach Erreichen des Colons kann Nifedipin in ausreichendem Maße resorbiert werden. Im Dickdarm ist jedoch zu berücksichtigen, daß einerseits wegen der geringen vorherrschenden Flüssigkeitsströme die Wirkstoffauflösung, andererseits unmittelbar damit verbunden auch die Diffusion innerhalb des weiten Darmlumens bis zur Darmwand zu ausgeprägten geschwindigkeitsbestimmenden Schritten werden können. Deshalb sollte gerade in diesem Darmabschnitt die Freisetzung und Auflösung des Wirkstoffs trotz der geringen Flüssigkeitsströme,gewährleistet sein. Dies ist bei herkömmlichen Retardformulierungen meistens nicht der Fall.

Damit wird besonders deutlich, daß, wie Untersuchungen bei Nifedipin, wie auch für andere Dihydropyridinderivate ergeben haben, nicht der gesamte Gastrointestinaltrakt für die Resorption zur Verfügung steht. Darüberhinaus kann die Resorption auch starken interindividuellen Schwankungen unterworfen sein.

Daher wird heute z.T. in Frage gestellt, ob der perorale Applikationsweg für Retardarzneiformen mit verzögerter Wirkstofffreigabe für alle Wirkstoffe überhaupt sinnvoll ist.

Eine Hauptvoraussetzung für die Erzielung konstanter Plasmaspiegel, nämlich die kontinuierliche Resorption von freigesetztem Wirkstoff aus der Arzneiform, scheint so nicht gegeben bzw. kann mit konventioneller Galenik nicht gewährleistet werden.

Die FR-A-2 608 427 beschreibt Hydrosole von pharmakologisch aktiven Substanzen und diese entaltende pharmazeutische Zubereitungen, bei denen die pharmakologisch aktiven Substanzen in Form von Hydrosolen vorliegen. Jedoch bilden die pharmazeutischen Zubereitungen injizierbare Präparate.

J.J. Marty et al., Pharm. Acta Helv. 53, 1 (1978) S. 17-23 beschreibt die Herstellung von Gelatine-Nanopartikeln, in die auch Wirkstoffe eingeschlossen werden können. Bei der Herstellung dieser Gelatine-Nanopartikel wird zur Desolvatation und Resolvatation eine pH-Justierung vorgesehen. Eine Überführung des Arzneimittels in Nanopartikel wird nicht offenbart.

Der vorliegenden Erfindung liegt daher die Aufgabe zugrunde, eine Arzneiform für die Stoffgruppe der Dihydropyridine, insbesondere Nifedipin bereitzustellen, die für retardierte Arzneistofffreigabe geeignet ist und die die Probleme des Standes der Technik überwindet.

Diese Aufgabe wird erfindungsgemäß dadurch gelöst, daß das Retard-Arzneimittel das pharmakologisch wirksame Dihydropyridinderivat in Form eines pharmazeutisch applizierbaren Nanosols aufweist, das als Träger im wesentlichen Gelatine, fraktionierte Gelatine oder ein Gelatinederivat neben üblichen pharmazeutischen Hilfsstoffen enthält, wobei das Nanosol
a) eine innere Phase aus dem Dihydropyridinderivat, das eine Teilchengröße von 10 - 800 nm aufweist und eine Oberflächenladung besitzt,
b) eine äußere Phase aus Gelatine, fraktionierter Gelatine oder einem Gelatinederivat, welche(s) gegensinnig geladen ist, und
c) einen annähernden oder vollständigen isoionischen Ladungszustand der inneren und äußeren Phase aufweist, und
d) physiologisch resorbierbar ist.

Diese Aufgabe wird weiterhin durch ein Verfahren zur Herstellung eines kolloid-dispersen Systems eines Dihydropyridinderivates gelöst, das dadurch gekennzeichnet ist, daß man eine Gelatine, Gelatinederivat, fraktionierte Gelatine und/oder deren Mischungen nach ihrem (seinem) isoelektrischen Punkt (IEP) so auswählt, daß ihr (sein) IEP mit dem Ladungszustand der Partikel des Dihydropyridinderivatpartikels so abgestimmt ist, daß die Gelatine, fraktionierte Gelatine oder das Gelatinederivat bei einem bestimmten pH-Wert mit den ungelösten Partikeln des Dihydropyridinderivates zu Ladungsneutralität führt; die Gelatine, fraktionierte Gelatine oder das Gelatinederivat in die wäßrige Solform überführt; den pH-Wert in Abhängigkeit von dem IEP der Gelatine auf einen solchen Wert einstellt, daß die sich bildenden Nanopartikel des Dihydropyridinderivates annähernd oder vollständig ladungsneutral stabilisiert werden; und vor oder nach der letztgenannten Stufe das Dihydropyridinderivat in dem wäßrigen Gelatinesol löst oder eine Lösung des Dihydropyridinderivates mit dem wäßrigen Gelatinesol vereinigt.

Diese Aufgabe wird weiterhin durch ein Verfahren für ein gesteuert freisetzendes Matrix-System auf Gelatinebasis gelöst, gekennzeichnet durch den in einer sich in wäßrigem Medium oberhalb 37°C auflösenden Gelatinematrix verteilten Arzneistoff, die in der internationalen (PCT)-Patentanmeldung "Sol-gesteuerte Thermokolloidmatrix auf Gelatinebasis für perorale Retardformen" der ALFATEC-Pharma GmbH vom selben Anmeldetag, entsprechend der deutschen Patentanmeldung DE-A-41 40 192 offenbart ist.

Ausführungsformen der erfindungsgemäßen Arzneimittel, sowie des Verfahrens zu ihrer Herstellung und ihre Verwendung werden in den Unteransprüchen genannt und beansprucht.

Weitere Patentanmeldungen der ALFATEC-Pharma GmbH, gegebenenfalls auch der PAZ Arzneimittelentwicklungsgesellschaft mbH, von demselben Tage betreffen die Akutform von 2-Arylpropionsäurederivaten (DE-A- 41 40 185), die Akutform von S- und R-Ibuprofen (DE-A- 41 40 179), die Retardform von S- und R-Ibuprofen (DE-A- 41 40 172), die Akutform von S- und R-Flurbiprofen (DE-A- 41 40 184), die Retardform von S- und R-Flurbiprofen (DE-A- 41 40 183), die Retardform von 3-Indolylessigsäurederivaten (DE-A- 41 40 191) und die o.g.Anmeldungen (DE-A- 41 40 195 und DE-A- 41 40 192).

Fig. 1 zeigt die Freigabe von Nifedipin gemäß Beispiel 1.

Fig. 2 zeigt die Freigabe von Nifedipin gemäß Beispiel 1.

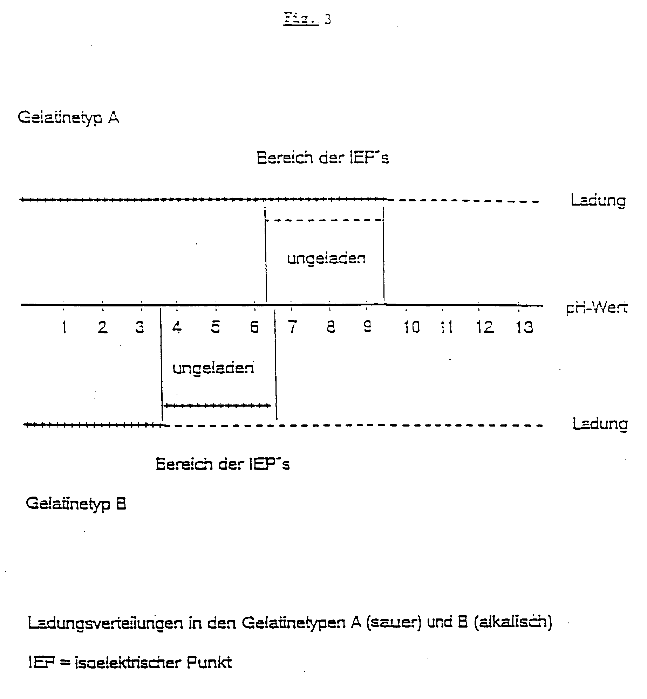

Fig. 3 zeigt eine schematische Darstellung der einstellbaren Ladungszustände von Gelatine in Abhängigkeit vom pH-Wert und IEP, wobei der IEP je nach Herstellungsart zwischen 3,5 und 9,5 liegen kann. Unterhalb von pH 3,5 sind fast alle Gelatinetypen positiv geladen. Im basischen Bereich oberhalb von pH 9,5 sind alle Gelatinetypen negativ geladen.

Fig. 4 zeigt den Mechanismus der passiven Arzneistoff-Resorption im Gastrointestinal-Trakt.

Erfindungsgemäß sind bedeutende Vorteile zu erzielen:
Überraschenderweise zeigt sich, daß das Vorliegen stabiler Nanopartikel im Falle der schwer wasserlöslichen Dihydropyridine, völlig ausreichend ist, eine Arzneistoffresorption im gesamten Gastrointestinaltrakt (GIT) -auch im Magen- zu erreichen, die
a) unabhängig von den oben geschilderten physiologischen Bedingungen ist
b) unabhängig von den physikalisch-chemischen Eigenschaften der Dihydropyridine ist
c) nahezu vollständig ist und
d) ohne vorgelagertes Gleichgewicht der Wirkstoffauflösung erfolgt wie bei herkömmlichen Retardformen, da der Wirkstoff in resorptionsfähiger Form unmittelbar an jedem beliebigen Resorptionsort zur Verfügung steht.

Offensichtlich werden Nanosole nach einem bisher nicht bekannten Mechanismus resorbiert.

Betrachtet man nun die gastrointestinale Passage der in Nanosolform vorliegenden Retardformulierung, die beispielsweise ein Nifedipin-Nanosol enthält, das während dieser konstant freigegeben wird, so ergibt sich ein völlig neuartiges Bild.

Unmittelbar auf die Freigabe der Nanopartikel aus der Arzneiform im Magen erfolgt deren Resorption. Es ist dabei gleichgültig, wie lange die Formulierung selbst im Magen verweilt. Schwankende Magenverweilzeiten von z.B. singleunit Retardarzneiformen müssen nicht mehr berücksichtigt werden, wie bei konventioneller Galenik. Damit wird ein Wirkungseintritt nach Applikation zeitlich mit hoher Genauigkeit vorhersagbar.

Die Verwendung von Gelatine zur Nanosolbildung und gleichzeitiger Verwendung als Matrixsubstanz bietet zusätzliche Vorteile, da eine gleichmäßige Verteilung der eingebetteten, erfindungsgemäßen Nanopartikel auf der Schleimhautoberfläche kontinuierlich im Retardierungszeitraum gewährleistet ist.

Somit ist die erfindungsgemäße Einbettung der Nanosole in einer retardierenden Gelatinematrix zusätzlich auch wirksam vor den Einflüssen von Nahrung und Nahrungsbestandteilen geschützt.

Um die physiologischen Hintergründe der Resorption von Arzneistoffen im allgemeinen und die verbesserte Resorptionsquote der erfindungsgemäßen Nanosole ausreichend zu erläutern, ist zunächst eine Betrachtung zum Mechanismus der physiologischen Resorption von Arzneistoffen erforderlich, wie er auch in einschlägigen Publikationen dargestellt wird. Allerdings ist die vorliegende Erfindung weder an den folgenden Versuch einer wissenschaftlichen Erklärung der erfindungsgemäß auftretenden Phenomene gebunden noch kann sie hierdurch eingeschränkt werden.

Die passive Arzneistoffresorption erfolgt nach heutigem Erkenntnisstand (Theorie nach Brodie et al.), wenn folgende Bedingungen vorliegen:
a) die Gastrointestinalmembran wirkt als Lipidbarriere,
b) der Arzneistoff wird nur in gelöster und ungeladener, d.h. nichtionisierter Form aufgenommen,
c) saure Arzneistoffe werden bevorzugt im Magen, basische Arzneistoffe bevorzugt im Darm resorbiert.

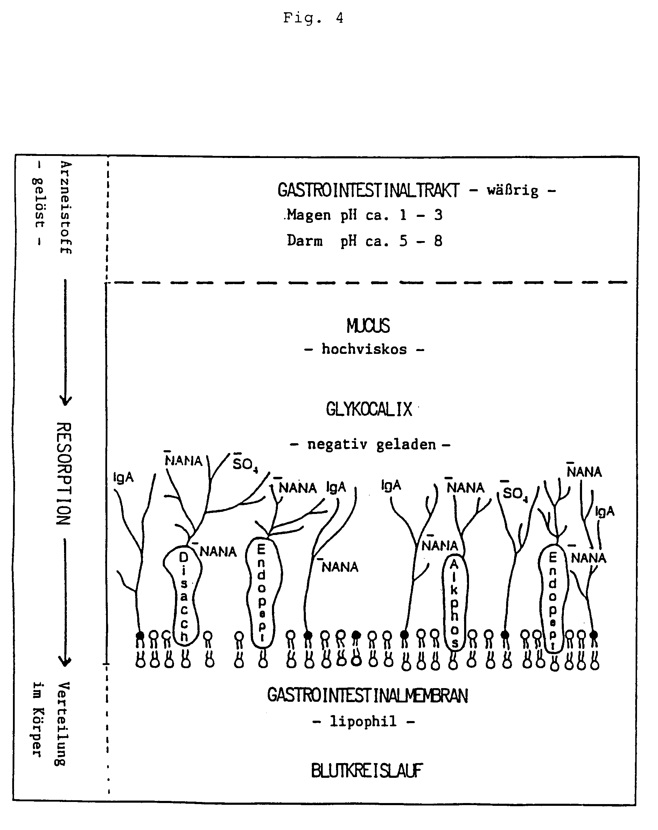

Nach der peroralen Aufnahme eines Arzneistoffs in den Organismus wird seine Resorption, d.h. der Übertritt in den allgemeinen Kreislauf (Biophase) in starkem Maße durch physikalische Barrieren behindert (siehe Abb. 4), nämlich
- durch die Mucus-Schicht und eine wässerige, daran adhärierende Schicht
- die Zellmembranen der intestinalen Epithelzellen mit der daran kovalent gebundenen Glykocalix sowie
- die sogenannten "Tight Junctions", die die Epithelzellen an ihrer apikalen Seite miteinander verbinden.

Diese Barrieren bedingen, daß die Resorption von Arzneistoffen hauptsächlich abhängig von ihrem Verteilungsmechanismus und Ladungszustand- durch die Lipid-Doppelschichten erfolgt (sogenannte passive Diffusion).

Die Epithelzellen des gesamten Magen-Darm-Traktes sind mit einer Mucus-Schicht bedeckt, die aus Mucinen (Glykoproteinen), Elektrolyten, Proteinen und Nucleinsäuren besteht. Vor allem die Glykoproteine bilden mit dem Hauptanteil des Mucus, nämlich Wasser, eine viskose Gelstruktur, die in erster Linie Schutzfunktionen für die darunter liegende Epithelschicht ausübt. Die Mucusschicht ist an die apikale Oberfläche der Epithelzellen über die Glykocalix gebunden. Die Glykocalix hat ebenfalls eine Glykoproteinstruktur, die kovalent an Bausteine der Membran-Doppelschicht der Epithelzellen gebunden ist. Die verzweigten Polysaccharide der Glykocalix, die entweder direkt an amphiphile Moleküle der Doppelmembran oder an die Doppelmembran inkorporierte Proteine kovalent gebunden sind, besitzen geladene N-Acetyl-Neuraminsäure- und Sulfat-Reste und sind daher negativ geladen, was zu einer elektrostatischen Bindung oder Abstoßung von geladenen Arzneistoffmolekülen bzw. von elektrostatisch geladenen Partikeln führen kann. Die Epithelzellmembranen bestehen aus Phospholipid-Doppelschichten, in die Proteine über ihre hydrophoben Bereiche verankert sind. Die Phospholipid-Doppelschichten mit ihrem lipophilen Anteil stellen eine weitere Barriere für den Transport der zu resorbierenden Arzneistoffe dar.

Aus dieser Darstellung geht deutlich hervor, daß geladene Arzneistoffmoleküle bzw. elektrostatisch geladene Partikel daher nur eine sehr geringe Chance haben, über den peroralen Applikationsweg resorbiert zu werden.

Die erfindungsgemäßen Nanosole geben erstmalig die technische Lehre, ein System zu bilden, mit dem diese vorgenannten Resorptionshindernisse zu überwinden sind. Da die Wirkstoff-Nanopartikel durch die Gelatine erfindungsgemäß ladungsneutral stabilisiert werden, kann ihr Transport durch die negativ geladene Glykocalix ohne größere Hindernisse erfolgen, im Gegensatz zu sonstig beschriebenen Nanopartikeln des Standes der Technik, die nicht ladungsneutral stabilisiert werden bzw. stabilisiert werden können. Erfindungsgemäß kann die Einstellung des isoionischen Ladungszustandes zusätzlich noch in Abstimmung auf die physiologischen Verhältnisse erfolgen (siehe insbesondere die Ausführungen auf S. 9, Zeile 14-20 in Verbindung mit Beispiel 1 und 2).

Da die erfindungsgemäßen Wirkstoff-Nanosole die Glykocalix ungehindert passieren können, ohne durch elektrostatische Effekte gebunden bzw. abgestoßen zu werden, erreichen sie damit auch die Oberfläche der Epithelzellen und stehen dort in hoher Konzentration zur Verfügung.

Nun können auch aktive, carriervermittelte Transportmechanismen bzw. Phagozytose einen wesentlichen Beitrag zur Resorption der Wirkstoff-Nanosole liefern.

Während der Magenpassage bis hin zum Dickdarm ist keine Einschränkung der kontinuierlichen Resorption der Nanopartikel in den erfindungsgemäßen Nanosolen zu befürchten.

Es hat sich nämlich gezeigt, daß auch in den verschiedenen Darmabschnitten die Gelatine in den erfindungsgemäßen Nanosolen die gleichmäßige Verteilung der Nanopartikel auf der Darmschleimhaut ermöglicht. Durch diese "bioadhäsive" Wirkung kann auch im flüssigkeitsarmen Colon eine von den dort vorherrschenden physiologischen Bedingungen unabhängige Resorption der erfindungsgemäßen Nanopartikel gewährleistet werden.

Die Verarbeitung der erfindungsgemäßen Dihydropyridin-Nanosole kann mit konventionellen galenischen Retardierungsmethoden erfolgen.

Bei der Formulierung von Akut- bzw. Retardpräparaten macht der Pharmazeut einen grundsätzlichen Unterschied zwischen:
1. galenischer Zubereitung, d.h. einer Freisetzung des Arzneistoffes, z.B. aus einer Tablette in zeitlich schneller (Akutform) oder verlangsamter (Retardform) Weise;
und
2. dem arzneistoffspezifischen Resorptionsort, wie z.B. Magen oder bestimmte Darmabschnitte.

Die erfindungsgemäßen Nanosole sind in der Lage, unabhängig von der galenischen Zubereitung, aufgrund ihrer speziellen Zusammensetzung, im gesamten gastrointestinalen Bereich resorbiert zu werden. Sie können daher vorteilhaft zu Akut- bzw. Retardarzneiformen weiterverarbeitet werden.

Als besondere Ausführungsform der vorliegenden Erfindung ist die in der schon genannten Patentanmeldung mit dem Titel "Sol-gesteuerte Thermokolloidmatrix auf Gelatinebasis für perorale Retardformen" beschriebene Retardformulierung bevorzugt geeignet. Eine solche Arzneiform setzt die Dihydropyridinderivate mit hoher Reproduzierbarkeit kontinuierlich frei und vermeidet die bekannten Nebenwirkungen. Die Gelatinematrix schützt weiterhin vor physiologisch bedingten pH-Schwankungen, die die Stabilität der Nanosole beeinträchtigen kann.

Durch alle die genannten Vorzüge gemeinsam lassen sich bei Dihydropyridinderivaten, insbesondere Nifedipin, Bioverfügbarkeiten erreichen, wie sie bisher nicht bekannt sind. Damit verbunden ist ebenso eine Verkürzung der Zeit von der Applikation bis zum Erreichen der Plasmawirkstoffkonzentration im therapeutischen Niveau (steady-state), als auch eine geringe Schwankungsbreite des Plasmaspiegels. Außerdem wird die in der erfindungsgemäßen Arzneiform enthaltene Wirkstoffdosis vollständig ausgenutzt, sodaß damit insgesamt gesehen eine Dosisverminderung gegenüber konventionellen Retardformen bei vergleichbarer Wirkung zustande kommt.

Erstaunlicherweise hat sich gezeigt, daß die Nanopartikel in dem erfindungsgemäßen Nanosol an jedem gewünschten Resorptionsort ungehindert die Gastrointestinalmembran passieren können (resorbiert werden). Sie verhalten sich also, biopharmazeutisch gesehen, wie eine echte Lösung, ohne aber eine solche zu sein.

Für peroral anzuwendende Retardformen von diesen sogenannten Calciumantagonisten ist bisher nichts derartiges bekannt.

Wie in den eingangs aufgeführten Patentanmeldungen bereits beschrieben ist, gilt auch für die Dihydropyridinderivate erstaunlicherweise, daß nur Nanopartikel, deren Größe unter 800 nm liegt, bevorzugt im Bereich unterhalb von 600 nm vollständig und schnell resorbiert werden können. Diese Bedingungen werden durch die erfindungsgemäßen Nanosole, mit Dihydropyridinderivaten, insbesondere Nifedipin als Wirkstoff erfüllt.

Die Herstellung des erfindungsgemäßen Nanosols erfolgt völlig analog zu den in der o.g. internationalen (PCT)-Anmeldung (81AL2730) beschriebenen Verfahren und angegebenen Vorgehensweisen.

Die Herstellung der Matrixformulierung erfolgt analog der in der internationalen (PCT)-Patentanmeldung "Retardform für ein Ibuprofen enthaltendes Arzneimittel und seine Herstellung" (81AL2734) bzw. der in der internationalen (PCT)-Patentanmeldung "Sol-gesteuerte Thermokolloidmatrix auf Gelatinebasis" (81AL2737) erläuterten Herstellungsweisen.

Prinzipiell sind zur Herstellung der erfindungsgemäßen Nanosole die in der o.g. deutschen Patentanmeldung P 41 40 195.6 der ALFATEC-Pharma GmbH "Pharmazeutisch applizierbares Nanosol und Verfahren zu seiner Herstellung" genannten Vorgehensweisen und Verfahrensvarianten geeignet, die im folgenden noch einmal angeführt werden:

Es werden mehrere Verfahren zur Herstellung der Nanosole vorgeschlagen. Dabei handelt es sich um eine beispielhafte, unvollständige Aufzählung. Der Fachmann kann aufgrund seines Fachwissens selbstständig weitere Varianten im Rahmen der vorliegenden Erfindung ausarbeiten:

### Verfahren I

Dieses kann angewendet werden, wenn der Arzneistoff in einer Mischung aus:
einem mit Wasser mischbaren organischen Lösungsmittel und Wasser, oder
aus mehreren mit Wasser mischbaren organischen Lösungsmitteln und Wasser
löslich ist:
a) eine in den Vorversuchen ausgewählte Gelatine wird mit Wasser in Solform überführt;
b) der in den Vorversuchen gefundene pH-Wert der Lösung wird eingestellt;
c) ein oder mehrere mit Wasser mischbare(s), organische(s) Lösungsmittel, vorzugsweise Ethanol, Isopropanol oder Methanol, wird/werden zu dieser Lösung gegeben;
d) der Arzneistoff wird in fester Form zu der Lösung gegeben und gelöst;
e) das/die organische(n) Lösungsmittel wird/werden entfernt, vorzugsweise durch Eindampfen in Vakuum; dabei entsteht das Nanosol;
f) die kolloid-disperse Lösung wird anschließend, vorzugsweise durch Sprüh- oder Gefriertrocknung, getrocknet.

Das organische Lösungsmittel hat die Aufgabe, den Arzneistoff zu lösen und verändert auch die Hydrathülle der Gelatinemoleküle.

### Verfahren II

Diese Ausführungsform kann angewendet werden, wenn der Arzneistoff eine Säure oder eine Base ist, deren Salz in Wasser löslich ist:
a) eine in den Vorversuchen ausgewählte Gelatine wird mit H₂O in die Solform überführt;
b) es wird ein solcher pH-Wert eingestellt, der die Salzbildung des Arzneistoffs ermöglicht;
c) der Arzneistoff wird unter Salzbildung in dem Gelatinesol gelöst;
d) durch Zugabe von Alkohol oder ähnlichen organischen Lösungsmitteln kann die Hydrathülle der Gelatinemoleküle gelockert werden;
e) durch Zugabe einer geeigneten Menge Säure oder Base wird der pH-Wert eingestellt, der zur Bildung des isoionischen Punkts (IIP) führt, dabei entsteht das Nanosol;
f) die kolloid-disperse Lösung wird wie in Verfahren I getrocknet.
Stufe d) ist fakultativ, jedoch bevorzugt.

### Verfahren III

Diese Ausführungsform kann angewendet werden, wenn der Arzneistoff ein Neutralstoff ist:
a) es wird ein Gelatinesol hergestellt, wie unter (1) a) und b) beschrieben.
b) eine zweite Lösung aus einem mit Wasser mischbaren organischen Lösungsmittel, vorzugsweise Ethanol, Methanol, Isopropanol, Aceton und dem Arzneistoff wird hergestellt.
c) die beiden Lösungen werden vereinigt.
d) das organische Lösungsmittel wird entfernt und die kolloid-disperse Lösung wird getrocknet.

### Verfahren IV

a) Wie unter (I) a) und b) beschrieben.
b) In einer zweiten Lösung wird ein kolloid-disperses System mit dem Arzneistoff kurzzeitig gebildet, jedoch ohne Gelatine.
c) Die unter (b) erhaltene Lösung wird kontinuierlich mit der Gelatinelösung vereinigt.

Bei Schritt (IV) c) kann die kontinuierliche Vermischung der unter (IV) a) und b) beschriebenen Lösungen zeitabhängig durch on-line Messung der Teilchengröße mit einem geeigneten Verfahren, wie z.B. durch Laser-Licht-Streuung (BI-FOQELS On-line Particle Sizer), gesteuert werden. Damit ist es möglich, eine gewünschte Partikelgröße kontinuierlich einzustellen.

Alle genannten Verfahren sind auch für Kollagenhydrolysate und Gelatinederivate geeignet und können problemlos in den technischen Maßstab übertragen werden.

Die wesentlichen Schritte können weitgehend automatisiert ablaufen, wobei auch Verfahren I bis III kontinuierlich durchführbar sind. Im Falle der Akutform für 2-Arylpropionsäurederivate seien als bevorzugt geeignete Verfahren die Varianten Nr. II und III genannt.

Für die erfindungsgemäßen Akutformen eignen sich alle Gelatinen, Gelatinederivate, Kollagenhydrolysate und fraktionierte Gelatine, sowie deren Mischungen. Gelatinesorten, die einen erfindungsgemäß beschriebenen isoelektrischen Punkt (IEP) aufweisen, der nicht handelsüblich ist, können nach den Beispielen I bis III aus o.g. deutscher Patentanmeldung hergestellt werden.

Gegenüber handelsüblichen Produkten führt die Verwendung von Gelatine, die auf spezielle Weise hergestellt wurde, zu erfindungsgemäß beschriebenen Nanosolen mit erhöhter Stabilität.

Beispiele für die Herstellung erfindungsgemäß besonders geeigneter Gelatinequalitäten werden unten gegeben.

### Beispiele für die Herstellung von erfindungsgemäß besonders geeigneten Gelatinesorten mit isoelektrischen Punkten von 3,5 bis 9,5

### Beispiel I:

### Verfahren zur Erzielung eines IEP's von 7,5 bis 9,5

Kollagenhaltiges Ausgangsmaterial wie z.B. Schweineschwarten werden mit einer wäßrigen Lösung einer 0,45 N Mineralsäure, vorzugsweise Schwefelsäure, im Flottenverhältnis 1:1 12 bis 20 Stunden behandelt. Anschließend wird der Säureüberschuß durch mehrmaliges Waschen entfernt, wobei zur Abkürzung des Verfahrens Natriumhydrogencarbonat verwendet werden kann. Die Extraktion des sudreifen Materials erfolgt mit heißem Wasser bei 55 - 80° C bei einem pH von 2,5 bis 4,5. Bei pH-Werten unterhalb von 3,5 kann ein IEP von 8,5 bis 9,5 erreicht werden, bei pH-Werten oberhalb 3,5 liegt der IEP bei 7 bis 8,5. Auf diese Weise lassen sich verschiedene IEP's von 7 bis 9,5 in direkter Abhängigkeit vom pH-Wert während der Extraktion erzielen.

Nach der Verfahrensstufe der Extraktion wird die wäßrige Lösung neutralisiert und wie üblich aufgearbeitet.

Durch dieses Verfahren kann man weiterhin in Abhängigkeit von der gewählten Temperatur während der Extraktion Gelatinesorten mit hohen bis mittleren Molekulargewichtsverteilungen erhalten.

Bei Temperaturen von 50-55° C erhält man besonders hochviskose und hochbloomige Qualitäten. Gelatinesorten mit niedrigem Molekulargewicht bzw. kaltwasserlösliche Gelatinen können durch gezielten Abbau mit Kollagenasen erhalten werden.

### Beispiel II:

### Verfahren zur Erzielung eines IEP's von 4 bis 7,5

Das kollagenhaltige Ausgangsmaterial wird zur Entfernung von Fremdstoffen zunächst gewaschen, zerkleinert und anschließend durch Zusatz von Magnesit, Natronlauge oder Calciumhydroxid durch gründliches Vermischen im Flottenverhältnis 1:1,2 homogen alkalisch gemacht. Das so vorbehandelte Material wird kurzzeitig druckhydrolytisch bei 1,01 x 10⁵ bis 2,02 x 10⁵ Pa und einem pH-Wert der wäßrigen Lösung von 8-14 aufgeschlossen. Nach dem Aufschluß wird sofort neutralisiert und die noch heiße wäßrige Gelatinelösung wie üblich filtriert, entsalzt, aufkonzentriert und getrocknet.

Nimmt man ein schwach basisches Aufschlußmittel wie Magnesit, erhält man einen IEP von 6 bis 7,5, sofern man bei 1,01 x 10⁵ Pa arbeitet. IEP's von 5 bis 6 erhält man bei Einsatz einer verdünnten Kalkmilchsuspension und bei Verwendung von 0,005 bis 0,1 N Natronlauge können IEP's von 4 bis 5 erzielt werden.

Gelatinesorten mit geringem Racemisierungsgrad und niedrigem Peptidanteil lassen sich bei Druckverhältnissen von 1,01 x 10⁵ Pa und Verweilzeiten von maximal 10 Min. erreichen.

Mittel- bis niedrigmolekulare bis hin zu kaltwasserlöslichen Sorten ergeben sich durch entsprechend längere Verweilzeiten.

### Beispiel III:

### Verfahren zur Erzielung eines IEP's von 3,5 bis 6

Kollagenhaltiges Ausgangsmaterial, vorzugsweise Spalt bzw. Ossein, wird nach der Eingangswäsche einem Kurzzeitäscher unterworfen. Hierbei bieten sich zwei Verfahrensvarianten im Flottenverhältnis 1:1,3 an, die entweder eine gesättigte Kalkmilchsuspension oder eine 0,1 bis 1 N Natronlauge zum Einsatz bringen.

Bei Verwendung einer Kalkmilchsuspension wird das Rohmaterial unter ständiger Bewegung maximal 3 bis 4 Wochen aufgeschlossen. Anschließend wird das Material durch Säurezugabe neutralisiert und mehrmals gewaschen. Die weitere Aufarbeitung folgt wie üblich. Auf diese Weise lassen sich IEP's von 4 bis 6 einstellen.

Bei Einsatz von Natronlauge läßt sich der Äscherprozeß nochmals verkürzen, wobei bei Konzentrationen von 1 N Natronlauge das Material je nach Zerkleinerungsgrad bereits nach 6 - 12 Stunden aufgeschlossen ist. Die Neutralisation erfolgt mit äquimolaren Mengen Mineralsäure und die Neutralsalze werden durch mehrmaliges Waschen oder durch Entsalzen der in der Extraktion gewonnenen wäßrigen Gelatinelösung entfernt. Bei dieser Verfahrensvariante lassen sich IEP's von 3,5 bis 5 erhalten.

Besonders peptidarme Gelatinesorten werden bei kurzer Verweilzeit im Äscher erhalten. Man kann so Gelatinesorten mit hoher bis mittlerer Molekulargewichtsverteilung (M = 10⁴ - 10⁷ D) erhalten.

Niedrigmolekulare bis kaltwasserlösliche Gelatinesorten kann man durch thermischen Abbau bzw. enzymatisch erhalten.

Bevorzugt werden im Falle der 2-Arylpropionsäurederivate Gelatinesorten mit IEP von 3,5 bis 9,5 eingesetzt.

Übliche pharmazeutische Hilfsstoffe und/oder weitere Makromoleküle können, sofern sie technologisch erforderlich sind, in flüssigem oder getrocknetem Zustand den erfindungsgemäßen Nanosolen zugesetzt werden.

Zum Beispiel kann ein Zusatz von Polyvinylpyrrolidon im Mengenverhältnis Gelatine zu Polyvinylpyrrolidon im Bereich von 5:1 bis 500:1 geeignet sein.

Eine Akutform im Sinne der Erfindung, die z.B. zu Tabletten verarbeitet wird oder lyophilisiert werden soll, kann durch Zusatz von niedrigmolekularen Polyvinylpyrrolidonsorten im Bereich von 10:1 bis 50:1 in den technologischen Verarbeitungseigenschaften verbessert werden, ohne daß die Stabilität der Nanosole negativ beeinflußt wird.

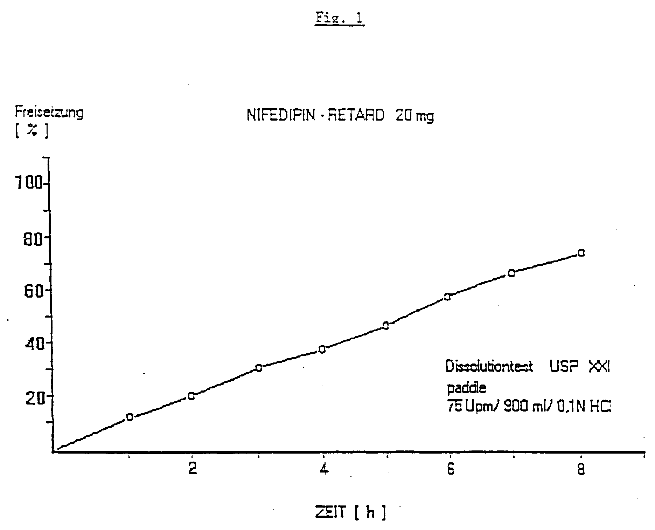

Die in den folgenden Beispielen bevorzugten Herstellungsverfahren, Vorgehensweisen und Bezeichnungen beziehen sich wie folgt auf die deutschen Patentanmeldungen "Pharmazeutisch applizierbares Nanosol und Verfahren zu seiner Herstellung" (P 41 40 195.6) bzw. die oben genannten Verfahren und Beispiele:
Nanosol-Herstellung: Verfahren II und III
Gelatineherstellung: Beispiel I bis III
Vortest: siehe folgende Beschreibung:
Vortest:
Wie eingangs schon erwähnt und wie aus Fig.1 ersichtlich ist, hängt die absolute, maximal mögliche Nettoladung eines einzelnen Gelatinemoleküls hauptsächlich von der Anzahl der freien COOH- und NH₂-Gruppen und dem pH-Wert der Lösung ab. Da sich Typ A, B, Kollagenhydrolysate oder Gelatinederivate in der Anzahl freier COOH-Gruppen unterscheiden, ist damit auch ihre maximal mögliche Nettoladung unterschiedlich. Bei Gelatinederivaten kann der Ladungszustand zusätzlich von der Art der Modifizierung abhängen.

Bei der Durchführung des erfindungsgemäßen Verfahrens wählt man in einem Vortest die geeignete Gelatine und den geeigneten pH-Wert aus.

Zunächst wird ein den physikalisch-chemischen Eigenschaften des Arzneistoffs angepaßter Arbeits-pH-Bereich gewählt. Als physikalisch-chemische Eigenschaft des Arzneistoffs sind vor allem zu berücksichtigen: Die Löslichkeit (in organischen Lösungsmitteln bzw. Wasser), seine Eigenschaft als Säure, Base oder Neutralstoff sowie seine Stabilität gegenüber Säuren und Laugen.

In einem ersten Schnelltest wird festgestellt, welche Ladung die ausgefällten Partikel besitzen. Daraus ergibt sich, unter Berücksichtigung des Arbeits-pH-Bereichs, die Wahl eines geeigneten Gelatinetyps. Sind die Teilchen beispielsweise negativ geladen, sucht man eine Gelatine aus, die unter den gegebenen pH-Bedingungen positiv geladen ist. Dieser Schnelltest zur Feststellung der Partikelladung hat die Vorteile, daß er ohne großen apparativen und zeitlichen Aufwand durchgeführt werden kann. So kann auf eine zeitaufwendige und ungenaue Zeta-Potential-Messung gänzlich verzichtet werden.

In vielen Fällen wird es ausreichend sein, für diesen Schnelltest zwei handelsübliche Gelatinen Typ A und B mit einem IEP von 9,5 bzw. 3,5 mit Peptidanteilen <30 % und einer Bloomzahl von 200, die weiterhin als Standardgelatinen bezeichnet werden, bei einem pH-Wert von 6 in die Solform zu überführen (5%ige wäßrige Lösung) und den Arzneistoff in einem mit Wasser mischbaren Lösungsmittel, wie z. B. Ethanol, Isopropanol oder Aceton, zu lösen und jeweils mit den Gelatinelösungen homogen zu mischen. Bei gleicher Dosierung des Arzneistoffs wird sich bei der in ihrem Ladungszustand nicht geeigneten Gelatine ein kolloidales System entweder nicht ausbilden oder sofort instabil werden bzw. der Arzneistoff ausflocken. Sind die entstehenden Partikel negativ geladen, werden sie eher von Gelatinelösung mit Typ A, der bei einem pH-Wert von 6 positiv geladen ist, stabilisiert als von der Lösung mit Gelatine Typ B; im Gegenteil wird in diesem Fall Typ B entweder kein kolloidales System ausbilden oder das System sofort destabilisieren. Das Ausflocken der Teilchen läßt sich z. B. über eine einfache Trübungs-Messung verfolgen.

Bei diesem Schnelltest muß auf jeden Fall der Arbeits-pH-Bereich beachtet werden. Man kann auch andere Gelatinen als Standard auswählen, sie müssen jedoch in ihrem IEP so gewählt werden, daß sie bei diesem pH-Wert entgegengesetzte Nettoladung tragen (siehe auch Fig.1). In den meisten Fällen werden die besagten Standardgelatinen Typ A und B für diesen Schnelltest ausreichen.

Ausgehend vom Ergebnis des Vorversuchs werden nun durch schrittweise Variation des IEP's durch Verwendung entsprechender Gelatinesorten und des pH-Wertes der Lösung in kleineren Bereichen (z. B. 0,1 pH-Schritte) die optimalen Bedingungen zur Bildung der Nanosole ermittelt. D.h. es muß das Stabilitätsoptimum, das durch den isoionischen Punkt (IIP) gekennzeichnet ist, gefunden werden, um eine ausreichende Stabilität für die genannten pharmazeutischen Anwendungen zu gewährleisten.

Es kann durchaus der Fall sein, daß eine im Sinne der Erfindung akzeptable Stabilität der Nanosole bereits in einem engeren pH-Bereich (ca. 0,5 Einheiten) um den isoionischen Punkt gefunden wird, so daß eine Einstellung dieses Punktes selbst nicht unbedingt notwendig ist. Andererseits können auch mehrere Gelatinen zu den gleichen, stabilen Ergebnissen führen. So kann beispielsweise (Beispiel 5) mit dem oralen Antidiabetikum Glibenclamid bei einem Gelatinetyp B mit einem IEP von 5,5 das Stabilitätsoptimum bei einem pH-Wert von 3,2 liegen, während bei einem Gelatinetyp B mit einem IEP von 3,8 das Stabilitätsoptimum bei einem pH-Wert von 2,2 liegt.

Gekennzeichnet durch ein Stabilitätsmaximum, wurde in beiden Fällen der isoionische Punkt erreicht (die Abhängigkeit der Nettoladung vom pH-Wert und dem IEP muß nicht linear sein, da sie durch den pKₛ-Wert der vorhandenen COOH- bzw. NH₃⁺ - Gruppen gegeben ist).

Erfindungsgemäß können alle Gelatinesorten mit einem Maximum der Molekulargewichtsverteilung im Bereich von 10⁴ bis 10⁷ D eingesetzt werden.

Kollagenhydrolysate, fraktionierte Gelatine mit niedrigem MG, Gelatinederivate und Gelatinen mit niedrigen Bloomwerten sind dann geeignet, wenn die so hergestellten Nanosole mit geeigneten galenischen Methoden unter Zusatz von weiteren Hilfsstoffen retardiert vorliegen.

Besonders geeignet sind Gelatinesorten mit einem Peptidanteil < 5% und einem Maximum der Molekulargewichtsverteilung oberhalb von 9,5 x 10⁴ D. Vorteilhaft können besonders hoch-viskose Gelatinesorten oder fraktionierte Gelatinen mit einem prozentualen Gewichtsanteil der Mikrogelfraktion (> 10⁷ D) größer als 15 Prozent eingesetzt werden.

Solche Gelatinen besitzen in weiten pH-Bereichen eine erhöhte Pufferkapazität und fördern durch ihre hochviskose Eigenschaft die Ausbildung eines physiologischen "Nanomilieus". Sie erhöhen damit die therapeutische Wirkung und Verträglichkeit im Sinne der Erfindung.

Durch Kombination der beschriebenen Vorgehensweise lassen sich Gelatinesorten finden, die technologisch gesehen auf überraschend einfache Weise zu Retardarzneiformen mit neuen Eigenschaften führen.

In Abhängigkeit von der Herstellungsweise von Gelatine (Ausmaß des Abbaus des nativen Kollagens und saures bzw. alkalisches Aufschlußverfahren) weist Gelatine vom Typ A oder Typ B ein charakteristisches Molekulargewichtsspektrum bzw. Molekulargewichtsverteilung auf. In Tabelle 1 sind die Molekulargewichtsverteilungen von verschiedenen Gelatinetypen bzw. von Kollagenhydrolysaten angegeben, sowie der prozentuale Anteil (Häufigkeit) einzelner Molekulargewichtsbereiche.

**Tabelle 1**

| Molekulargewichtsverteilung von verschiedenen bekannten Gelatinetypen bzw. von bekannten Kollagenhydrolysaten | | | | | | | |
|---|---|---|---|---|---|---|---|
| Molekulargewichtsverteilung (KD) | Natives Kollagen % | Gelatine Typ B % | Gelatine Typ A % | Kollagenhydrolysat Gelita®-Collagel A | Kollagenhydrolysat Gelita®-Collagel B | Kollagenhydrolysat Gelita®-Sol C | Elastinhydrolysat Gelita®-Gelastin |
| >360 | 100 | 18,0 | 18,0 | 0 | 0 | 0 | 0 |
| 285 | 0 | 7,0 | 9,0 | 0 | 0 | 0 | 0 |
| 145-237 | 0 | 20,0 | 34,0 | 1,0 | 1,5 | 0 | 0 |
| 95 | 0 | 25,0 | 11,0 | 0 | 0 | 0 | 0 |
| 95-50 | 0 | 16,3 | 13,4 | 2,5 | 4,0 | 1,1 | 0 |
| 50-20 | 0 | 7,4 | 9,1 | 18,0 | 14,5 | 0,3 | 0 |
| 20-10 | 0 | 3,9 | 3,8 | 43,0 | 31,5 | 3,7 | 0,2 |
| 10-5 | 0 | 3,0 | 3,0 | 15,4 | 20,0 | 12,2 | 5.2 |
| 5-2 | 0 | 0 | 0 | 6,0 | 14,0 | 26,0 | 93,9 |
| 2-1 | 0 | 0 | 0 | 7,0 | 8,0 | 23,0 | 0 |
| <1 | 0 | 0 | 0 | 6,5 | 7,0 | 34,0 | 0 |
| MG | 360 | 165 | 185 | 12 - 18 | 12 - 18 | 3 | 2-3 |

Man erkennt in den einzelnen Spalten deutlich das Überwiegen eines einzelnen Bereiches im Vergleich zu den übrigen Molekulargewichtsbereiechen derselben Gelatine. Dieser Bereich stellt also das Maximum der Molekulargewichtsverteilung dar (es liegt z.B. bei der in der Abbildung aufgeführten Gelatine Typ B bei 95 kD). Der Begriff des "Maximums der Molekulargewichtsverteilung" ist jedoch streng zu trennen von dem Begriff des "durchschnittlichen mittleren Molokulargewichts". Dieser Mittelwert liegt bei der erwähnten Gelatine vom Typ B bei 165 kD.

Als Verhältnis von Gelatine zu Wirkstoff können für die Erfindung bevorzugt 3:1 bis 20:1 gewählt werden.

Die erfindungsgemäßen Nanosole können beispielsweise sprühgetrocknet, gefriergetrocknet und im trockenen Zustand wieder resuspendiert werden.

Erstaunlicherweise lassen sich die erfindungsgemäßen, getrockneten Nanosole leicht zu einer pharmazeutischen Zubereitung verarbeiten, die die Dihydropyridinderivate eingebettet in eine Matrix enthalten. Nach der Applikation sind die erfindungsgemäßen Nanopartikel in der sich ausbildenden, dünnen Solschicht wirksam vor physiologischen Einflüssen geschützt Darüberhinaus bleibt im Innern der Arzneiform stets ein trockener, fester Kern vorhanden. Solche Matrixarzneiformen zeichnen sich auch durch eine gewisse Haftwirkung an Schleimhautoberflächen aus. Das spielt besonders im Dickdarm eine große Rolle, weil die Flüssigkeitsströme in diesem Darmabschnitt im Vergleich zu anderen GIT-Abschnitten sehr viel geringer sind. Die im Stand der Technik beschriebenen, herkömmlichen Wirkstoffauflöse- und Diffusionsprozesse, die wiederum die Wirkstoffresorption beeinflussen können entfallen somit.

Als Applikationsformen eignen sich Tabletten bzw. komprimierte Pellets. Vorteilhaft können Akutformen auf Nanosolbasis mit Retardtabletten z.B. in Hartgelatinekapseln kombiniert werden, wobei für die Akutform eine Dosierung von 5 mg meist genügt. Eine Dihydropyridin-Akutform ist in der Internationalen (PCT)-Anmeldung 81AL2730 beschrieben.

Die in den Beispielen gewählten Dosierungen sind für eine Einmalgabe (24 h) geeignet.

Weiterhin bietet die Einbettung dieser Wirkstoffklasse in Gelatine einen optimalen Schutz vor Lichteinwirkung, die für diese Wirkstoffgruppe besonders wichtig ist. Dieser Schutz kann noch verstärkt werden, wenn man der Gelatinelösung vor der Trocknung z.B. pharmazeutisch geeignete gelbe Farbstoffe zusetzt, oder eine Gelatinesorte mit ausgeprägter gelblicher Eigenfarbe auswählt.

Folgende Beispiele sollen die vorliegende Erfindung näher erläutern:
Alle Arbeiten mit Dihydropyridinen werden unter Lichtschutz (Gelblicht) durchgeführt.

Da Dihydropyridine chemisch gesehen Neutralstoffe sind, kann bevorzugt das oben beschriebene Verfahren III angewendet werden.

### Beispiel 1:

### a) Herstellung des Nifedipin-Nanosols

Wirkstoff: Nifedipin, Neutralstoff
Gelatinesorte: Typ B (IEP 4,7), 330 Bloom,
Herstellung: Beispiel II
Nansol-Herstellung: analog Verfahren III
Gewichtsverhältnis Gelatine/Arzneistoff: 20:1
Der Vortest und die anschließende Meßreihe ergibt ein Stabilitätsoptimum für eine Gelatine Typ B (IEP 4,7) bei einem pH-Wert von 5,5.

Eine Gelatinelösung aus 600 g oben spezifizierter Gelatine wird als 6%ige Lösung bei 60°C hergestellt. Der pH-Wert wird auf 5,5 eingestellt.

30 g Nifedipin werden in 0,5 l Isopropanol gelöst.

Die beiden Lösungen werden vereinigt, wobei sich das Nanosol bildet. Das organische Lösungsmittel wird unter Vakuum entfernt und die kolloiddisperse Lösung wird sprühgetrocknet.

Teilchengrößenmessungen ergeben durchschnittliche Partikelgrößen im Bereich von 310 - 350 nm.

### b) Herstellung der Nifedipin-Retardmatrix

Das unter a) erhaltene Pulver wird auf einer Exzenterpresse direkt zu Tabletten geformt, die jeweils einen Gehalt von 20 mg Nifedipin haben.

Der Dissolutiontest in einer Apparatur nach USP (paddle, 75 Upm/900 ml 0,1 N HCl) ergibt die in Fig.1 dargestellte Freigabe.

### Beispiel 2:

### a) Herstellung des Nifedipin-Nanosols

Wirkstoff: Nifedipin, Neutralstoff
Gelatinesorte: Typ B (IEP 4,7), 370 Bloom,
Mikrogel: 16 Gew.-%
Herstellung: Beispiel II
Nansol-Herstellung: analog Verfahren III
Gewichtsverhältnis Gelatine/Arzneistoff: 15:1
Eine Gelatinelösung aus 600 g oben spezifizierter Gelatine wird als 6%ige Lösung bei 60°C hergestellt. Der pH-Wert wird auf 5,5 eingestellt.

37,5 g Nifedipin werden in 0,5 l Isopropanol gelöst.

Die beiden Lösungen werden vereinigt, wobei sich das Nanosol bildet. Das organische Lösungsmittel wird unter Vakuum entfernt und die kolloiddisperse Lösung wird sprühgetrocknet.

Teilchengrößenmessungen ergeben durchschnittliche Partikelgrößen im Bereich von 280 - 310 nm.

### b) Herstellung der Nifedipin-Retardmatrix

Das unter a) erhaltene Pulver wird auf einer Exzenterpresse direkt zu Tabletten geformt, die jeweils einen Gehalt von 30 mg Nifedipin haben.

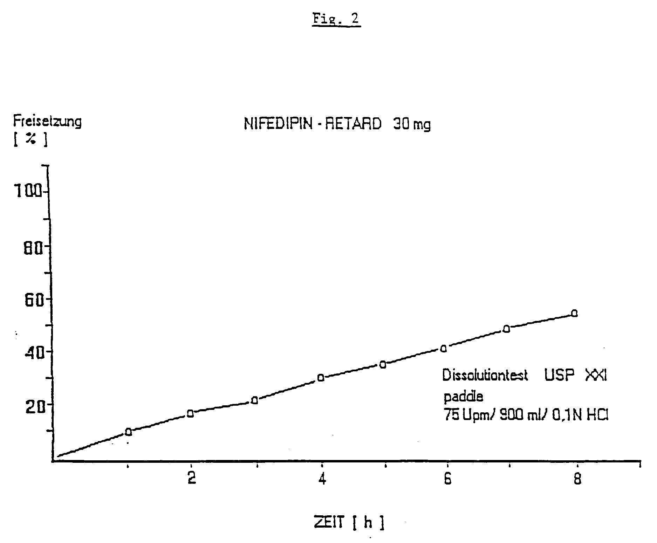

Der Dissolutiontest in einer Apparatur nach USP (paddle, 75 Upm/900 ml 0,1 N HCl) ergibt die in Fig.2 dargestellte Freigabe.

## Patentansprüche

1. Retard-Arzneimittel, enthaltend ein pharmakologisch wirksames Dihydropyridinderivat in Form eines pharmazeutisch applizierbaren Nanosols, das als Träger im wesentlichen Gelatine, fraktionierte Gelatine oder ein Gelatinederivat neben üblichen pharmazeutischen Hilfsstoffen enthält, wobei das Nanosol
a) eine innere Phase aus dem Dihydropyridinderivat, das eine Teilchengröße von 10 - 800 nm aufweist und eine Oberflächenladung besitzt,
b) eine äußere Phase aus Gelatine, fraktionierter Gelatine oder einem Gelatinederivat, welche(s) gegensinnig geladen ist, und
c) einen annähernden oder vollständigen isoionischen Ladungszustand der inneren und äußeren Phase aufweist, und
d) physiologisch resorbierbar ist.

2. Arzneimittel nach Anspruch 1 und/oder 2, dadurch gekennzeichnet, daß das Dihydropyridinderivat als feste, resuspendierbare Nanodispersion vorliegt.

3. Arzneimittel nach einem der Ansprüche 1 bis 3, dadurch gekennzeichnet, daß das Dihydropyridinderivat eine durchschnittliche Teilchengröße unterhalb von 400 nm aufweist.

4. Arzneimittel nach einem der Ansprüche 1 bis 3, dadurch gekennzeichnet, daß die Gelatine ein Maximum der Molekulargewichtsverteilung im Bereich von 10⁴ bis 10⁷ D aufweist.

5. Arzneimittel nach Anspruch 4, dadurch gekennzeichnet, daß die Gelatine ein Maximum der Molekulargewichtsverteilung oberhalb von 9,5 x 10⁴ D und einen Peptidanteil kleiner als 5 Gewichtsprozent aufweist.

6. Arzneimittel nach einem der Ansprüche 1 bis 5, dadurch gekennzeichnet, daß die Gelatine einen prozentualen Gewichtsanteil der Mikrogelfraktion (> 10⁷ D) größer als 15 % aufweist.

7. Arzneimittel nach einem der Ansprüche 1 bis 6, gekennzeichnet durch eine äußere Phase des Nanosols, die zusätzlich viskositätserhöhende Stoffe in einem Gewichtsverhältnis von Gelatine zu synthetischem oder natürlichem Polymer wie 10:1 bis 1000:1 enthält.

8. Arzneimittel nach einem der Ansprüche 1 bis 7, dadurch gekennzeichnet, daß die Retardform eine Tablette ist.

9. Arzneimittel nach Anspruch 8, dadurch gekennzeichnet, daß die Retardform eine Matrixtablette ist.

10. Arzneimittel nach einem der Ansprüche 1 bis 9, dadurch gekennzeichnet, daß das Dihydropyridinderivat als Retardmatrix auf Gelatinebasis in Form eines pharmazeutisch applizierbaren Nanosols vorliegt.

11. Arzneimittel nach einem der Ansprüche 1 bis 10, dadurch gekennzeichnet, daß das Dihydropyridinderivat teilweise als Akutform in Form eines pharmazeutisch applizierbaren Nanosols vorliegt.

12. Arzneimittel nach einem der Ansprüche 9 bis 11, dadurch gekennzeichnet, daß die Akut- und Retardform in einer Hartgelatinekapsel vorliegen.

13. Arzneimittel nach einem der Ansprüche 1 bis 12, dadurch gekennzeichnet, daß die äußere Phase gelb gefärbt ist.

14. Verfahren zur Herstellung eines kolloid-dispersen Systems eines Dihydropyridinderivates, dadurch gekennzeichnet, daß man
a) eine Gelatine oder ihr Derivat nach ihrem isoelektrischen Punkt (IEP) so auswählt, daß ihr IEP mit dem Ladungszustand der Dihydropyridinderivatpartikel so abgestimmt ist, daß die fraktionierte Gelatine oder ihr Derivat bei einem bestimmten pH-Wert mit dem ungelösten Dihydropyridinderivat zu annähernder oder vollständiger Ladungsneutralität führt,
b) die (fraktionierte) Gelatine oder ihr Derivat in die wäßrige Solform überführt,
c) den pH-Wert in Abhängigkeit von dem IEP der Gelatine auf einen solchen Wert einstellt, daß die sich bildenden Nanopartikel des Dihydropyridinderivates annähernd oder vollständig ladungsneutral stabilisiert werden, und
d) vor oder nach der Stufe c) das Dihydropyridinderivat in dem wäßrigen Gelatinesol löst oder eine Lösung des Dihydropyridinderivates mit dem wäßrigen Gelatinesol vereinigt.

15. Verfahren nach Anspruch 14, dadurch gekennzeichnet, daß man in Stufe d) das gelöste Dihydropyridinderivat vor der Vereinigung mit dem wäßrigen Gelatinesol in kolloid-disperse Form von Nanopartikeln überführt und die so erhaltene Dispersion von Nanopartikeln mit dem wäßrigen Gelatinesol vereinigt.

16. Verfahren nach Anspruch 14 oder 15, dadurch gekennzeichnet, daß man e1) das Dihydropyridinderivat in Form von Nanopartikeln ausfällt.

17. Verfahren nach Anspruch 14 oder 15, dadurch gekennzeichnet, daß man e2) die in Stufe d) erhaltene kolloid-disperse Lösung sprühtrocknet oder gefriertrocknet und so ein stabiles resuspendierbares Nanosol erhält, das nach Wiederauflösung in wäßrigem Medium ein kolloid-disperses System in Nanosolform ergibt.

18. Verfahren nach den Ansprüchen 14 bis 17, dadurch gekennzeichnet, daß man in Stufe d) das Dihydropyridinderivat in einem mit Wasser mischbaren organischen Lösungsmittel gelöst, zusetzt.

19. Verfahren nach einem der Ansprüche 14 bis 18, dadurch gekennzeichnet, daß man eine Gelatine mit gelber Eigenfarbe einsetzt oder der Gelatinelösung einen pharmazeutisch geeigneten gelben Farbstoff zusetzt.

20. Verfahren nach einem der Ansprüche 14 bis 18, dadurch gekennzeichnet, daß man die mit Wasser mischbaren organischen Lösungsmittel in Stufe b) dem wäßrigen Gelatinesol zusetzt und in Stufe d) das Dihydropyridinderivat in fester Form dieser Mischung zusetzt und damit löst.

21. Verfahren nach Anspruch 18 oder 20, dadurch gekennzeichnet, daß man das organische Lösungsmittel anschließend wieder entfernt.

22. Verfahren nach einem der Ansprüche 14 bis 16, dadurch gekennzeichnet, daß man die Nanopartikel-Lösung anschließend trocknet.

23. Verfahren nach Anspruch 22, dadurch gekennzeichnet, daß man die Lösung sprühtrocknet.

24. Verfahren nach Anspruch 22, dadurch gekennzeichnet, daß man die Lösung gefriertrocknet.

25. Verfahren nach einem der Ansprüche 14 oder 15, dadurch gekennzeichnet, daß man im Anschluß an Stufe d) ein mit Wasser mischbares organisches Lösungsmittel zur Lockerung der Hydrathülle der Gelatinemoleküle zusetzt.

26. Verfahren nach Anspruch 25, dadurch gekennzeichnet, daß man einen Alkohol zusetzt.

27. Verfahren nach Anspruch 15, dadurch gekennzeichnet, daß man die kolloiden Teilchen kontinuierlich mit einer einstellbaren Partikelgröße herstellt.

28. Verfahren nach Anspruch 27, dadurch gekennzeichnet, daß man die Teilchengröße kontinuierlich mißt.

29. Verfahren nach einem der Ansprüche 14 bis 28, dadurch gekennzeichnet, daß man es kontinuierlich durchführt.

30. Verwendung eines pharmazeutisch applizierbaren Nanosols von einem Dihydropyridinderivat, das als Träger im wesentlichen Gelatine, fraktionierte Gelatine oder ein Gelatinederivat neben üblichen pharmazeutischen Hilfsstoffen enthält, wobei das Nanosol
a) eine innere Phase aus dem Dihydropyridinderivat, das eine Teilchengröße von 10 - 800 nm aufweist und eine Oberflächenladung besitzt,
b) eine äußere Phase aus Gelatine, fraktionierter Gelatine oder einem Gelatinederivat, welche(s) gegensinnig geladen ist, und
c) einen annähernden oder vollständigen isoionischen Ladungszustand der inneren und äußeren Phase aufweist, und
d) physiologisch resorbierbar ist,
zur Herstellung eines Calciumantagonisten.

## Claims

1. Sustained-release medicament, containing a pharmacologically active dihydropyridine derivative in the form of a pharmaceutically administrable nanosol which as excipient essentially contains gelatin, fractionated gelatin or a gelatin derivative in addition to customary pharmaceutical auxiliaries, the nanosol
a) having an inner phase of the dihydropyridine derivative which has a particle size of 10 - 800 nm and possesses a surface charge,
b) an outer phase of gelatin, fractionated gelatin or a gelatin derivative, which is oppositely charged, and
c) an approximately or completely isoionic charge state of the inner and outer phase and
d) being physiologically absorbable.

2. Medicament according to Claim 1 and/or 2, characterized in that the dihydropyridine derivative is present as a solid, resuspendable nanodispersion.

3. Medicament according to one of Claims 1 to 3, characterized in that the dihydropyridine derivative has an average particle size of below 400 nm.

4. Medicament according to one of Claims 1 to 3, characterized in that the gelatin has a maximum in the molecular weight distribution in the range from 10⁴ to 10⁷ D.

5. Medicament according to Claim 4, characterized in that the gelatin has a maximum in the molecular weight distribution of above 9.5 × 10⁴ D and a peptide content of less than 5 per cent by weight.

6. Medicament according to one of Claims 1 to 5, characterized in that the gelatin has a per centage weight content of the microgel fraction (> 10⁷ D) of greater than 15 %.

7. Medicament according to one of Claims 1 to 6, characterized in that an outer phase of the nanosol which additionally contains viscosity-increasing substances in a weight ratio of gelatin to synthetic or natural polymer such as 10:1 to 1000:1.

8. Medicament according to one of Claims 1 to 7, characterized in that the sustained-release form is a tablet.

9. Medicament according to Claim 8, characterized in that the sustained-release form is a matrix tablet.

10. Medicament according to one of Claims 1 to 9, characterized in that the dihydropyridine derivative is present as a sustained-release matrix based on gelatin in the form of a pharmaceutically administrable nanosol.

11. Medicament according to one of Claims 1 to 10, characterized in that the dihydropyridine derivative is present partly as an immediate-effect form in the form of a pharmaceutically administrable nanosol.

12. Medicament according to one of Claims 9 to 11, characterized in that the immediate-effect and sustained-release forms are present in a hard gelatin capsule.

13. Medicament according to one of Claims 1 to 12, characterized in that the outer phase is coloured yellow.

14. Process for the preparation of a colloidally disperse system of a dihydropyridine derivative, characterized in that
a) a gelatin or its derivative is selected according to its isoelectric point (IEP) such that its IEP is coordinated with the charge state of the dihydropyridine derivative particles such that the fractionated gelatin or its derivative leads to almost or complete charge neutrality with the undissolved dihydropyridine derivative at a certain pH,
b) the (fractionated) gelatin or its derivative is converted into the aqueous sol form,
c) the pH is adjusted as a function of the IEP of the gelatin to a value such that the nanoparticles of the dihydropyridine derivative formed are almost or completely stabilized in a neutrally charged form, and
d) before or after stage c) the dihydropyridine derivative is dissolved in the aqueous gelatin sol or a solution of the dihydropyridine derivative is combined with the aqueous gelatin sol.

15. Process according to Claim 14, characterized in that in stage d) the dissolved dihydropyridine derivative is converted into the colloidally disperse form of nanoparticles before combination with the aqueous gelatin sol and the dispersion of nanoparticles thus obtained is combined with the aqueous gelatin sol.

16. Process according to Claim 14 or 15, characterized in that e1) the dihydropyridine derivative precipitates in the form of nanoparticles.

17. Process according to Claim 14 or 15, characterized in that e2) the colloidally disperse solution obtained in stage d) is spray-dried or freeze-dried and a stable resuspendable nanosol is thus obtained which after redissolution in aqueous medium yields a colloidally disperse system in nanosol form.

18. Process according to Claims 14 to 17, characterized in that the dihydropyridine derivative dissolved in a water-miscible organic solvent is added in stage d).

19. Process according to one of Claims 14 to 18, characterized in that a gelatin having a yellow intrinsic colour is employed or a pharmaceutically suitable yellow colourant is added to the gelatin solution.

20. Process according to one of Claims 14 to 18, characterized in that the water-miscible organic solvents are added to the aqueous gelatin sol in stage b) and in stage d) the dihydropyridine derivative is added to this mixture in solid form and thus dissolved.

21. Process according to Claim 18 or 20, characterized in that the organic solvent is subsequently removed again.

22. Process according to one of Claims 14 to 16, characterized in that the nanoparticle solution is subsequently dried.

23. Process according to Claim 22, characterized in that the solution is spray-dried.

24. Process according to Claim 22, characterized in that the solution is freeze-dried.

25. Process according to one of Claims 14 or 15, characterized in that following stage d) a water-miscible organic solvent is added to loosen the hydration shell of the gelatin molecules.

26. Process according to Claim 25, characterized in that an alcohol is added.

27. Process according to Claim 15, characterized in that the colloidal particles are prepared continuously with an adjustable particle size.

28. Process according to Claim 27, characterized in that the particle size is continuously measured.

29. Process according to one of Claims 14 to 28, characterized in that it is carried out continuously.

30. Use of a pharmaceutically administrable nanosol of a dihydropyridine derivative which as excipient essentially contains gelatin, fractionated gelatin or a gelatin derivative in addition to customary pharmaceutical auxiliaries, the nanosol
a) having an inner phase of the dihydropyridine derivative which has a particle size of 10 - 800 nm and possesses a surface charge,
b) an outer phase of gelatin, fractionated gelatin or a gelatin derivative, which is oppositely charged, and
c) an approximately or completely isoionic charge state of the inner and outer phase and
d) being physiologically absorbable.
for the production of a calcium antagonist.

## Revendications

1. Médicament à action retardée, contenant un dérivé de dihydropyridine pharmacologiquement active sous forme d'un nanosol pharmaceutiquement applicable, qui contient comme support principalement de la gélatine, de la gélatine fractionnée ou un dérivé de gélatine, à côté de substances auxiliaires pharmaceutiques classiques, dans lequel le nanosol présente
a) une phase interne du dérivé de dihydropyridine qui présente des diamètres de particules de 10 à 800 nm et qui possède une charge de surface,
b) une phase externe formée de gélatine, de gélatine fractionnée ou d'un dérivé de gélatine, qui porte une charge opposée,
c) un état de charge presque isoionique ou tout à fait isoionique des phases interne et externe, et
d) peut être résorbé physiologiquement.

2. Médicament suivant la revendication 1, caractérisé en ce que le dérivé de dihydropyridine se présente comme nano-dispersion solide pouvant être remise en suspension.

3. Médicamént suivant la revendication 1 et/ou la revendication 2, caractérisé en ce que le dérivé de dihydropyridine présente un diamètre moyen de particules inférieur à 400 nm.

4. Médicament suivant l'une des revendications 1 à 3, caractérisé en ce que la gélatine présente un maximum de répartition de poids moléculaire dans la plage de 10⁴ à 10⁷ D.

5. Médicament suivant la revendication 4, caractérisé en ce que la gélatine présente un maximum de dispersion de poids moléculaire au-dessus de 9,5 x 10⁴ D et une fraction de peptide inférieur à 5 % en poids.

6. Médicament suivant l'une des revendications 1 à 5, caractérisé en ce que la gélatine présente un pourcentage en poids de fraction de microgel (> 10⁷ D) supérieur à 15 %.

7. Médicament suivant l'une des revendications 1 à 6, caractérisé par une phase externe du nanosol qui contient en outre des substances élevant la viscosité dans un rapport en poids de la gélatine au polymère synthétique ou naturel de 10:1 à 1000:1.

8. Médicament suivant l'une des revendications 1 à 7, caractérisé en ce que la forme à action retardée est un comprimé.

9. Médicament suivant la revendication 8, caractérisé en ce que la forme à action retardée est un comprimé à matrice.

10. Médicament suivant l'une des revendications 1 à 9, caractérisé en ce que le dérivé de dihydropyridine, comme matrice à action retardée à base de gélatine, se présente sous forme d'un nanosol pharmaceutiquement applicable.

11. Médicament suivant l'une des revendications 1 à 10, caractérisé en ce que le dérivé de dihydropyridine se présente en partie comme forme à action rapide sous forme d'un nanosol pharmaceutiquement applicable.

12. Médicament suivant l'une des revendications 9 à 11, caractérisé en ce que la forme à action rapide et la forme à action retardée se présentent dans une capsule de gélatine dure.

13. Médicament suivant l'une des revendications 1 à 12, caractérisé en ce que la phase externe est colorée en jaune.

14. Procédé de production d'un système en dispersion colloïdale d'un dérivé de dihydropyridine, caractérisé en ce que :
a) on choisit une gélatine ou son dérivé d'après son point isoélectrique (PIE) de manière que son PIE concorde avec l'état de charge des particules de dérivé de dihydropyridine de manière que la gélatine fractionnée ou son dérivé conduise à la neutralité approximative ou totale de charge avec le dérivé de dihydropyridine non dissous pour une valeur déterminée de pH,
b) on fait prendre à la gélatine (fractionnée) ou à son dérivé la forme d'un sol aqueux,
c) on règle la valeur de pH en fonction du PIE de la gélatine à une valeur telle que les nano-particules de dérivé de dihydropyridine qui se forment soient stabilisées en atteignant presque ou en atteignant totalement la neutralité de charge, et
d) avant ou après l'étape c), on dissout le dérivé de dihydropyridine dans le sol aqueux de gélatine ou bien on réunit une solution du dérivé de dihydropyridine avec le sol aqueux de gélatine.

15. Procédé suivant la revendication 14, caractérisé en ce que dans l'étape d), le dérivé de dihydropyridine dissous est transformé en une forme en dispersion colloïdale de nano-particules avant la réunion avec le sol aqueux de gélatine et la dispersion de nano-particules ainsi obtenue est réunie avec le sol aqueux de gélatine.

16. Procédé suivant la revendication 14 ou 15, caractérisé en ce que
e1) on précipite le dérivé de dihydropyridine sous forme de nano-particules.

17. Procédé suivant la revendication 14 ou 15, caractérisé en ce que
e2) on sèche par pulvérisation ou par congélation la solution en dispersion colloïdale obtenue dans l'étape d) et on obtient ainsi un nanosol stable pouvant être remis en suspension, qui donne après redissolution en milieu aqueux un système en dispersion colloïdale sous forme de nanosol.

18. Procédé suivant les revendications 14 à 17, caractérisé en ce que dans l'étape d), le dérivé de dihydropyridine est ajouté à l'état dissous dans un solvant organique miscible à l'eau.

19. Procédé suivant les revendications 14 à 18, caractérisé en ce qu'on utilise une gélatine de propre couleur jaune ou bien on ajoute à la solution de gélatine un colorant jaune acceptable du point de vue pharmaceutique.

20. Procédé suivant l'une des revendications 14 à 18, caractérisé en ce qu'on ajoute les solvants organiques miscibles à l'eau dans l'étape b) au sol aqueux de gélatine et, dans l'étape b), on ajoute le dérivé de dihydropyridine sous la forme solide à ce mélange et on l'y dissout.

21. Procédé suivant la revendication 18 ou 20, caractérisé en ce qu'on chasse ensuite le solvant organique.

22. Procédé suivant l'une des revendications 14 à 16, caractérisé en ce qu'on sèche ensuite la solution de nano-particules.

23. Procédé suivant la revendication 22, caractérisé en ce qu'on sèche la solution par pulvérisation.

24. Procédé suivant la revendication 22, caractérisé en ce qu'on sèche la solution par congélation.

25. Procédé suivant l'une des revendications 14 ou 15, caractérisé en ce que, à la suite de l'étape d), on ajoute un solvant organique miscible à l'eau pour affaiblir l'enveloppe d'hydrate des molécules de gélatine.

26. Procédé suivant la revendication 25, caractérisé en ce qu'on ajoute un alcool.

27. Procédé suivant la revendication 15, caractérisé en ce qu'on produit les particules colloïdales en continu avec un diamètre de particules réglable.

28. Procédé suivant la revendication 27, caractérisé en ce qu'on mesure en continu le diamètre des particules.

29. Procédé suivant l'une des revendications 14 à 28, caractérisé en ce qu'il est mis en oeuvre en continu.

30. Utilisation d'un nanosol, pharmaceutiquement applicable, d'un dérivé de dihydropyridine qui contient, comme support, principalement de la gélatine, de la gélatine fractionnée ou un dérivé de gélatine, à côté des substances auxiliaires pharmaceutiques classiques, dans laquelle le nanosol
a) présente une phase interne formée d'un dérivé de dihydropyridine, qui présente un diamètre de particules de 10 à 800 nm et possède une charge de surface,
b) une charge externe formée de gélatine, de gélatine fractionnée ou d'un dérivé de gélatine, qui porte une charge opposée,
c) un état de charge presque isoionique ou tout à fait isoionique des phases interne et externe, et
d) peut être résorbé physiologiquement,
pour la préparation d'un antagoniste du calcium.
